# EUROPEAN PATENT SPECIFICATION

(11) **EP 0 955 925 B1**
(45) Date of publication and mention of the grant of the patent: **15.05.2002**
(21) Application number: 96907830.2
(22) Date of filing: 12.03.1996
(51) Int. Cl.: A61B 17/50

(54) **DEVICE FOR REMOVING TICKS FROM HUMANS AND ANIMALS**
VORRICHTUNG ZUM ENTFERNEN VON ZECKEN BEI MENSCHEN ODER TIEREN
DISPOSITIF POUR ENLEVER LES TIQUES PRESENTES SUR LES HUMAINS ET LES ANIMAUX

(30) Priority: 15.03.1995 SE 9500942; 06.06.1995 SE 9600940
(43) Date of publication of application: 17.11.1999
(73) Proprietor: Innotech Sweden AB, 590 41 Rimforsa (SE)
(72) Inventor: LAXVIK, Lars, S-590 41 Rimforsa (SE)
(74) Representative: Rosenquist, Per Olof
(86) International application number: SE9600316
(87) International publication number: WO9628102

(56) References cited:
- EP-A- 0 027 704
- AT-A- 378 116
- CH-A- 239 112
- US-A- 4 938 764
- US-A- 5 407 243

## Description

The present invention concerns a device for removing ticks from humans and animals.

Known arrangements of the above-mentioned type are tweezer-like instruments. However these instruments are difficult to use since considerable experience is required to use the instrument correctly without injuring the tick. Such injury to or pressure on the tick can give rise to tick-borne infections. Furthermore, document AT-A-378 116 discloses a device for removing ticks consisting in elongated sleeve with a gripping device (i.e. a pair of flexible and spaced apart legs activated by a spring) inserted therethrough. This device also has a pushbutton, for discharging the gripping devide against the effect of the spring, at the end of the elongated sleeve that is opposite to the gripping device.

An object of the present invention is to provide a device of the initially mentioned type by means of which a tick can be removed rapidly and reliably so eliminating the above-mentioned disadvantages. This object is achieved according to the invention in that the device comprises: a base part provided with two openings and a tip tapering at the openings; a thread of resilient material which passes through the openings and thus forms a loop outside the base part, the loop being arranged such that, under the effect of a helical spring disposed inside the base part, it is drawn in towards the openings, the loop outside the openings, under the drawing-in effect of the thread, gradually decreasing in size, and the spring being arranged to actuate the thread with such a force that a tick is not injured by the loop; and a rod of which one end is connected to the thread and of which the other end projects out of the base part at the opposite end to the tip and thus forms a pushbutton for discharging the thread loop against the effect of the spring.

The loop is preferably made of nylon thread as emerges from a particular feature of the invention.

A method of removing ticks from humans and animals using the device according to the invention is characterized in that, by exerting pressure on the pushbutton, the loop is ejected from the opening and is laid around the tick, and in that, when pressure is no longer exerted on the pushbutton, the loop can be drawn into the opening, whereupon the loop is rotated one turn and the tick releases its grip within approximately three seconds.

An embodiment of the device according to the invention is shown schematically in the enclosed drawing and will be explained in further detail in the following with reference to this drawing, in which:

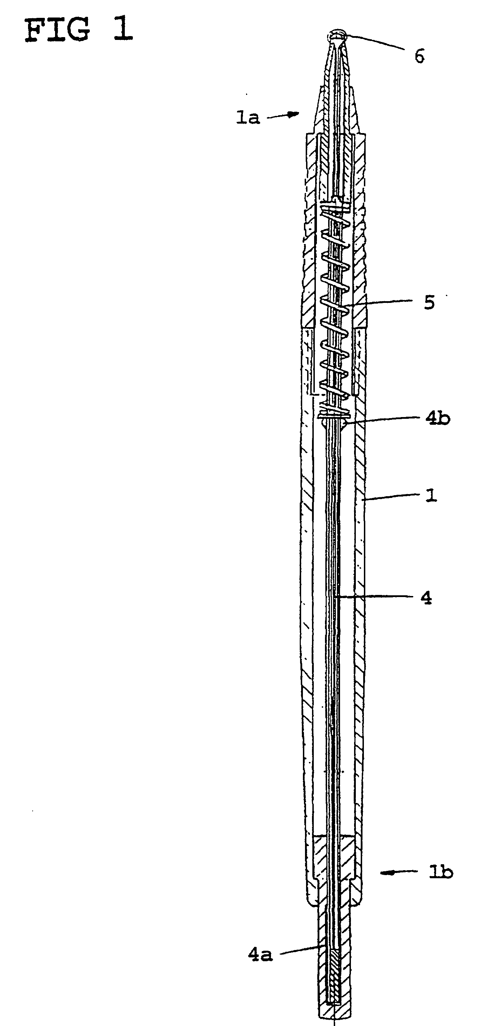

- Figure 1: shows schematically and in longitudinal section an embodiment of the device; and

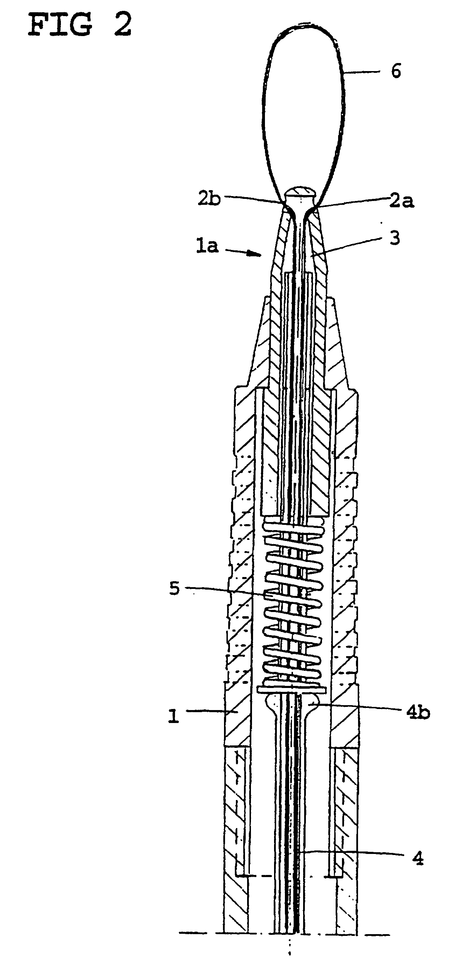

- Figure 2: shows a detail view of Figure 1, which shows clearly the loop before use.

In the drawings 1 designates a penholder-like base part which at its one conically tapering end 1a has two openings 2a, 2b which are widened to form a duct 3 inside the base part 1. Displaceably mounted in the duct 3 is a rod 4 which at the other end 1b of the base part 1 projects out the latter and forms a pushbutton 4a. When pressure is exerted on the pushbutton 4a, a helical spring 5, which is tensioned between a spring stop 4b on the rod 4 and the base of the conically tapering part 1a of the base part 1, counteracts this movement

Attached to the rod 4 at its end opposite the pushbutton 4a is a loop 6 of resilient material, for example, a nylon thread, which loop can be ejected from the interior of the base part 1, through the openings 2a, 2b owing to pressure being exerted on the pushbutton 4a.

The device according to the invention is used in the following way: the pushbutton 4a is pushed in against the effect of the spring 5, the loop 6 being ejected through the openings 2a, 2b. The loop 6 is passed over a tick which is to be removed and the pushbutton 4a is released so that the loop 6 is gradually drawn into the interior 3 of the base part 1. Owing to the effect of the spring 5, the tick is thus held fast by the loop 6 without being injured. The device and the loop are now rotated one turn clockwise or anticlockwise and within approximately three seconds the tick releases its grip and can be rendered harmless when it has been removed from the loop 6.

## Claims

1. Device for removing ticks from humans and animals, **characterized in that** the device comprises: a base part (1) provided with two openings (2a, 2b) and a tip tapering at the openings; a thread (6) of resilient material which passes through the openings and thus forms a loop (6) outside the base part (1), the loop being arranged such that, under the effect of a helical spring (5) disposed inside the base part, it is drawn in towards the openings (2a, 2b), the loop (6) outside the openings (2a, 2b), under the drawing-in effect of the thread, gradually decreasing in size. and the spring being arranged to actuate the thread with such a force that a tick is not injured by the loop (6); and a rod (4) of which one end is connected to the thread (6) and of which the other end projects out of the base part (1) at the opposite end to the tip and thus forms a pushbutton for discharging the thread loop (6) against the effect of the spring (5).

2. Device according to Claim 1, **characterized in that** the loop (6) is made of polyamide.

## Patentansprüche

1. Vorrichtung zum Entfernen von Zecken von Menschen and Tieren, **dadurch gekennzeichnet, dass** die Vorrichtung aufweist: ein Sockelteil (1), das mit zwei Öffnungen (2a, 2b) und einer sich an den Öffnungen verjüngenden Spitze versehen ist; einen Faden (6) aus flexibelem Material, der durch die Öffnungen hindurchtritt und somit eine Schleife (6) außerhalb des Sockelteils (1) ausbildet, wobei die Schleife derart eingerichtet ist, dass sie, unter der Wirkung einer im Inneren des Sockelteils (1) angeordneten Schraubenfeder (5), in Richtung der Öffnungen (2a, 2b) gezogen wird, wobei die Schleife (6) außerhalb der Öffnungen (2a, 2b), unter der Einzieh-Wirkung des Fadens, graduell ihre Größe verringert und wobei die Feder derart eingerichtet ist, dass eine solche Kraft auf den Faden ausgeübt wird, dass eine Zecke mittels der Schleife (6) nicht verletzt wird; und einen Stab (4), von dem ein Ende mit dem Faden (6) verbunden ist und von dem das andere Ende aus dem Sockelteil (1) aus dem der Spitze gegenüberliegenden Ende hervorsteht und somit einen Druckknopf zum Entlasten der Fadenschleife (6) gegen die Wirkung der Feder (5) ausbildet.

2. Vorrichtung gemäß Anspruch 1, **dadurch gekennzeichnet, dass** die Schleife (6) aus Polyamid hergestellt ist.

## Revendications

1. Dispositif pour enlever des tiques sur des êtres humains et des animaux, **caractérisé en ce que** le dispositif comprend: une partie de base (1) dotée de deux orifices (2a, 2b) et d'une pointe s'effilant en cône à hauteur des orifices; un fil (6) de matériau souple, qui passe à travers les orifices et forme ainsi une boucle (6) à l'extérieur de la partie de base (1), la boucle étant mise en place de telle sorte que sous l'action d'un ressort hélicoïdal (5) disposé à l'intérieur de la partie de base, elle soit rétractée en direction des orifices (2a, 2b), la boucle (6), à l'extérieur des orifices (2a, 2b), diminuant progressivement de taille sous l'action de rétraction du fil, et le ressort étant conçu pour agir sur le fil avec une force telle, qu'une tique ne soit pas blessée par la boucle (6); et une tige (6) dont une extrémité est reliée au fil (6) et dont l'autre extrémité fait saillie hors de la partie de base (1), à l'extrémité opposée à la pointe, et forme ainsi un bouton poussoir permettant de détendre la boucle de fil (6) à l'encontre de l'action du ressort (5).

2. Dispositif selon la revendication 1, **caractérisé en ce que** la boucle (6) est constituée de polyamide.
